(19) Europäisches Patentamt
European Patent Office
Office européen des brevets

(11) EP 4 015 650 A1

(12) EUROPEAN PATENT APPLICATION

(43) Date of publication:
22.06.2022 Bulletin 2022/25

(21) Application number: 20215730.1

(22) Date of filing: 18.12.2020

(51) International Patent Classification (IPC):
C12Q 1/6881 $^{(2018.01)}$    C12Q 1/6886 $^{(2018.01)}$
G16B 30/00 $^{(2019.01)}$

(52) Cooperative Patent Classification (CPC):
C12Q 1/6886; C12Q 1/6881; G16B 30/10;
G16B 40/20; G16H 50/30; C12Q 2600/156

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: NIPD GENETICS BIOTECH LIMITED
2409 Engomi Nicosia (CY)

(72) Inventors:
• KOUMBARIS, George
  2565 Lithrodontas (CY)
• ACHILLEOS, Achilleas
  4045 Limassol (CY)

• ELIADES, Alexia
  2102 Nicosia (CY)
• LOIZIDES, Charalambos
  2013 Nicosia (CY)
• TSANGARAS, Kyriakos
  4040 Limassol (CY)
• KYPRI, Elena
  2232 Nicosia (CY)
• IOANNIDES, Marios
  2416 Nicosia (CY)
• PATSALIS, Philippos
  2402 Nicosia (CY)

(74) Representative: CH Kilger Anwaltspartnerschaft
mbB
Fasanenstraße 29
10719 Berlin (DE)

(54) METHODS FOR CLASSIFYING A SAMPLE INTO CLINICALLY RELEVANT CATEGORIES

(57)    The disclosure provides methods and kits for the classification of biological samples into clinically relevant categories. The method comprises the steps of (i) determining in a sample comprising a plurality of cell-free DNA (cfDNA) fragments the sequence coordinates of the start and/or stop of at least 100,000 cfDNA fragments by alignment to a reference sequence, and (ii) determining in the reference sequence all nucleic acid motifs comprised of trinucleotides, tetranucleotides and pentanucleotides. Said nucleotides are: (a) within the range of 1 to 5 base pairs inwards but adjacent to each start and/or stop sequence coordinate determined in (i), and/or (b) within a range of 1 to 5 base pairs outwards but adjacent to each start and/or stop sequence coordinate determined in (i). Subsequently, in third step (iii) the frequency of (a) each sequence coordinate plus and/or minus 1 base pair determined in (i) in the plurality of cfDNA fragments comprised in the sample and (b) each of the nucleic acid motifs determined in (ii) a) and b) in the plurality of cfDNA fragments comprised in the sample is determined. Using these frequencies, the ratio of each of the frequencies determined in (iii) a) and b) over a corresponding reference frequency is calculated. A diagnostic score is calculated separately for each ratio mentioned above. As a final step, a combined diagnostic score from at least two or more diagnostic scores determined is calculated. The diagnostic scores and the combined diagnostic score allow for the classification of the sample into clinically relevant categories and the combined diagnostic score further allows for the classification of cell-free and/or circulating tumor DNA in the sample into low, moderate and high amount.

Figure 1

EP 4 015 650 A1

**Description**

**FIELD OF THE INVENTION**

The invention is in the field of biology, medicine and chemistry, in particular in the field of molecular biology and more in particular in the field of molecular diagnostics.

**BACKGROUND OF THE INVENTION**

Eukaryotic genomes are organized into chromatin, which enables not only to compact DNA but also regulates DNA metabolism (replication, transcription, repair, recombination). It has been shown that signatures of chromatin structure in eukaryotic organisms, in particular the nucleosome arrangement, can be used to identify rare nucleic acid fragments in complex mixtures present in eukaryotic organisms (Heitzer E. et al., Nat. Rev. Genet., 2019, 20(2):71-88).

The protection of the DNA by the nucleosome is hypothesized to be responsible for the presence of hot spots of non-random fragmentation (HSNRF) which are defined as regions in the genome where the ends of nucleic acid fragments of a specific size distribution are found to occur at a higher frequency than expected, when compared to nearby genomic locations.

Cancer is often found in non-easily accessible locations of the human body. The "gold standard" invasive surgical biopsies for the diagnosis of cancer impose significant clinical risks including bleeding and infection. Among the disadvantages of such invasive procedures is the fact that the sample taken from the tumor tissue is only a spatially limited representation from the time the procedure took place. Cancers, however, do not stay static but they undergo continuous changes that result in genetic heterogeneity within the tumor and between the primary and metastatic cancers. There has been great effort in developing non-invasive/minimally invasive methods for cancer diagnosis, monitoring and therapy guidance. The successful technological development of non-invasive prenatal testing of numerical abnormalities using cell free DNA from maternal plasma, could also be used for biomarker discovery, for the diagnosis of cancer. The discovery of circulating tumor DNA in plasma has provided the possibility to utilize it as a biomarker and employ liquid biopsy testing for the detection, prognosis, and prediction of response to cancer treatments, without having to address the risks associated with the invasive surgical procedures. This technology benefits cancer patients by detecting cancer at its early stages thus increasing the possibility of a successful recovery, aiding selection of the most appropriate therapy and also helps detecting minimal residual disease after the course of treatment, thus aiding clinicians in making necessary medical interventions. Unlike current invasive testing methods which have a risk of complications, liquid biopsy is inherently safe to the patient as it uses samples such as blood, urine or sputum.

To date, only a limited number of methods have been described which attempt to provide an estimate of the tumor-derived contribution to the total amount of cell free DNA (cfDNA) found in plasma, said cell free tumor DNA (cftDNA) to be used as prognostic biomarker, indicator of response and/or resistance to therapy and recurrence of disease (Smith C.G. et al., Genome Med., 2020, 12(1): 23; Peiyong Jiang et al., PNAS, 2018, 115(46): E10925-E10933; Cristiano S. et al. Nature, 2019, 570: 385-389; Mouliere et al., Sci. Transl. Med., 2018, 10(466): eaat4921; Newman A. et al., Nat. Med., 2014, 20(5): 548-554).

Current liquid biopsy- based tests fail to meet the needs of precision oncology because of their complexity as well their limited sensitivity and specificity (De Rubis G. et al., Trends Pharmacol Sci., 2019, 40(3): 172-186; Peiyong Jiang et al., Cancer Discov., 2020, CD-19-0622). Thus, the accuracy of such methods is not high enough and could give rise to misleading results.

The current invention provides a solution to the limitations faced by state-of-the-art liquid biopsy approaches by expanding the range of information extractable from circulating tumor DNA (ctDNA) sequencing and implementing novel multiparameter strategies to establish a robust, sensitive and specific liquid biopsy assay for the classification of samples into clinically relevant categories.

**SUMMARY OF THE INVENTION**

The current invention provides a solution to the accuracy limitations currently faced by other liquid biopsy approaches. The current invention overcomes said accuracy limitations by expanding the range of information extractable from cell-free tumor DNA or ctDNA sequencing and implementing novel multiparameter strategies to establish a robust, sensitive and specific liquid biopsy assay for the classification of samples into clinically relevant categories.

In one embodiment the present invention relates to a method of classifying a sample as comprising cell-free tumor DNA, the method comprising the steps of:

(i) determining in a sample comprising a plurality of cell-free DNA (cfDNA) fragments, the sequence coordinates of the start and/or stop of at least 100,000 cfDNA fragments by alignment to a reference sequence,

(ii) determining in the reference sequence all nucleic acid motifs comprised of trinucleotides, tetranucleotides and pentanucleotides:

    a) within the range of 1 to 5 base pairs inwards but adjacent to each start and/or stop sequence coordinate determined in (i), and/or

    b) within a range of 1 to 5 base pairs outwards but adjacent to each start and/or stop sequence coordinate determined in (i),

(iii) determining the frequency of:

a) each sequence coordinate plus and/or minus 1 base pair determined in (i) in the plurality of cfDNA fragments comprised in the sample,
b) each of the nucleic acid motifs determined in (ii) a) and b) in the plurality of cfDNA fragments comprised in the sample,

(iv) calculating the ratio of each of the frequencies determined in (iii) a) and b) over a corresponding reference frequency,
(v) calculating a diagnostic score separately for each ratio determined in step (iv), said score being the respective weighted sum of all respective frequency ratios of step (iv)
(vi) calculating a combined diagnostic score from at least two or more of the diagnostic scores determined in (v) said score being the weighted sum of said two or more diagnostic scores determined in (v), and
(vii) determining a classification of the sample by comparing the combined diagnostic score to a reference score,

wherein the sample is classified as comprising tumor cfDNA, if the combined diagnostic score value is higher than the mean of the reference score by at least one standard deviation of the reference score, wherein the reference score is calculated from one or more reference values.

In one embodiment the combined diagnostic score is calculated from all of the diagnostic scores calculated for each ratio calculated in step (v) of the method above.

In one embodiment the present invention relates to a method of classifying a sample as comprising cell-free tumor DNA, the method comprising the steps of:

(i) determining in a sample comprising a plurality of cell-free DNA (cfDNA) fragments the sequence coordinates of the start and/or stop, and of the start and/or stop plus and/or minus 1 base pair, of at least 100,000 cfDNA fragments by alignment to a reference sequence,
(ii) determining the frequency of each coordinate determined in (i) in the plurality of cfDNA fragments comprised in the sample,
(iii) calculating the ratio of the frequency of each coordinate determined in (ii) over a corresponding reference frequency,
(iv) calculating a diagnostic score from all ratios determined in (iii) said score being the weighted sum of all frequency ratios determined in (iii), and
(v) determining a classification of the sample by comparing the diagnostic score to a reference score,

wherein the sample is classified as comprising tumor cfDNA, if the diagnostic score value is higher than the mean of the reference score by at least one standard deviation of the reference score, wherein the reference score is calculated from one or more reference values.

In one embodiment the present invention relates to a method of classifying a sample as comprising cell-free tumor DNA, the method comprising the steps of:

(i) determining in a sample comprising a plurality of cell-free DNA (cfDNA) fragments the sequence coordinates of the start and/or stop of at least 100,000 cfDNA fragments by alignment to a reference sequence,
(ii) determining in the reference sequence all nucleic acid motifs comprised of trinucleotides, tetranucleotides and pentanucleotides within the range of 1 to 5 base pairs inwards but adjacent to each start and/or stop sequence coordinate determined in (i),
(iii) determining the frequency of each of the nucleic acid motifs determined in (ii) in the plurality of cfDNA fragments comprised in the sample,
(iv) calculating the ratio of each of the frequencies determined in (iii) over a corresponding reference frequency,
(v) calculating a diagnostic score from all ratios determined in (iv) said score being the weighted sum of all frequency ratios determined in (iv), and
(vi) determining a classification of the sample by comparing the diagnostic score to a reference score,

wherein the sample is classified as comprising tumor cfDNA, if the diagnostic score value is higher than the mean of the reference score by at least one standard deviation of the reference score, wherein the reference score is calculated from one or more reference values.

In another embodiment the present invention relates to a method of classifying a sample as comprising cell-free tumor DNA, the method comprising the steps of:

(i) determining in a sample comprising a plurality of cell-free DNA (cfDNA) fragments the sequence coordinates of the start and/or stop of at least 100,000 cfDNA fragments by alignment to a reference sequence,
(ii) determining in the reference sequence all nucleic acid motifs comprised of trinucleotides, tetranucleotides and pentanucleotides within the range of 1 to 5 base pairs outwards but adjacent to each start and/or stop sequence coordinate determined in (i),
(iii) determining the frequency of each of the nucleic acid motifs determined in (ii) in the plurality of cfDNA fragments comprised in the sample,
(iv) calculating the ratio of each of the frequencies determined in (iii) over a corresponding reference frequency,
(v) calculating a diagnostic score from all ratios determined in (iv), said score being the weighted sum of all frequency ratios determined in (iv), and

(vi) determining a classification of the sample by comparing the diagnostic score to a reference score,

wherein the sample is classified as comprising tumor cfDNA, if the diagnostic score value is higher than the mean of the reference score by at least one standard deviation of the reference score, wherein the reference score is calculated from one or more reference values.

In one embodiment the range of base pairs inwards but adjacent to each start and/or stop sequence coordinate can be from 2 bp to 6 bp, or 3 bp to 7 bp, or 4 bp to 8 bp, or 5 bp to 9 bp or 6 bp to 10 bp from each start and/or stop coordinate.

In one embodiment the minimum amount of cfDNA fragments comprised within a sample to be analyzed is between 100 thousand to 500 thousand, 500 thousand to 1 million, 1 million to 2 million, 2 million to 5 million, or 5 million to 10 million, or 10 million to 20 million, or 20 million to 50 million, or 50 million to 500 million.

In one embodiment the amount of tumor cfDNA in the sample can be classified as low if the combined diagnostic score is between 2 and 4 standard deviations of the reference scores, as moderate if the combined score is between 4 and 6.5 standard deviations of the reference scores and high if the combined score is more than 6.5 standard deviations of the reference scores.

In one embodiment the reference samples can be samples from cancer free patients, or from non-relapsed patients, or from successfully treated cancer patients.

In one embodiment the step (i) of any of the methods described above, of determining in a sample comprising a plurality of cell-free DNA (cfDNA) fragments the sequence coordinates of the start and/or stop of at least 100,000 cfDNA fragments by alignment to a reference sequence,, comprises the determination of the nucleic acid sequence of at least a portion of the plurality of cfDNA fragments in the sample prior to the alignment to a reference sequence.

In one embodiment the step (i) of any of the methods described above, of determining in a sample comprising a plurality of cell-free DNA (cfDNA) fragments the sequence coordinates of the start and/or stop of at least 100,000 cfDNA fragments by alignment to a reference sequence, further comprises the enrichment of cfDNA fragments prior to the determination of the nucleic acid sequence of cfDNA fragments.

In one embodiment the sample is classified as comprising tumor cfDNA originating from a tumor selected from the group of blood cancer, liver cancer, lung cancer, pancreatic cancer, prostate cancer, breast cancer, gastric cancer, glioblastoma, colorectal cancer, head and neck cancer, a solid tumor, a benign tumor, a malignant tumor, an advanced stage of cancer, a metastasis or a precancerous tissue.

In another embodiment the present invention relates to a kit comprising:

(i) components for carrying out any of the above described methods wherein the components comprise:

a) one or more components for isolating cell-free DNA from a biological sample,

b) one or more components for preparing and enriching the sequencing library, and/or

c) one or more components for amplifying and/or sequencing the enriched library,

(ii) software for performing statistical analysis.

## BRIEF DESCRIPTION OF THE FIGURES

Twenty normal samples from cancer-free patients and 27 abnormal samples from patients diagnosed with advanced Non-small-cell lung carcinoma (NSCLC) or colon cancer were analyzed. Ten randomly selected normal samples and ten randomly selected abnormal samples were used at the training step to estimate the unknown parameters in Examples 1-4.

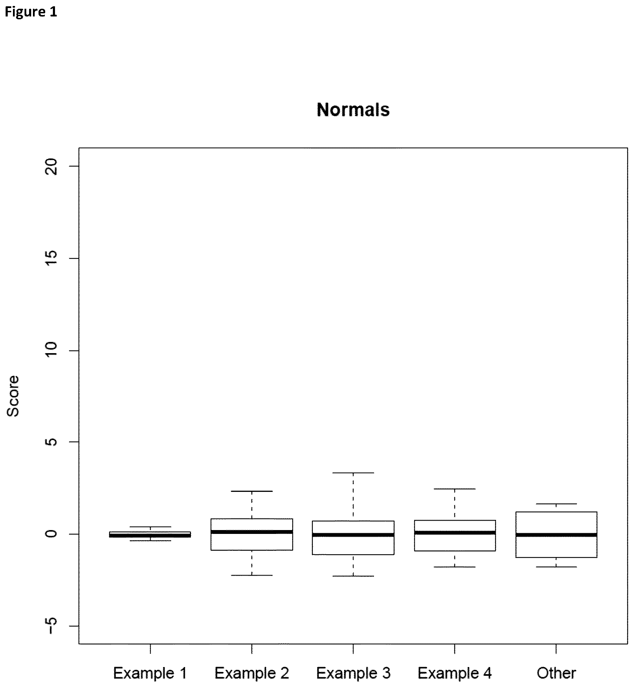

Figure 1: The figure shows the distribution of the scores obtained in Examples 1-4 for "normal" samples (control samples of healthy, cancer-free individuals not included in the training step) compared to the scores obtained by the method described in the state-of-the-art, hereby termed as "other" method (Peiyong Jiang et al., Cancer Discov., 2020, CD-19-0622). Said other method measuring the quantities of sequence end motifs of cfDNA fragments comprised in the samples analyzed, taking also into account and including the start and/or stop coordinates of said fragments, unlike the present disclosure, which excludes said start and/or stop. A non-significant Kruskal-Wallis rank sum test (p-value = 0.9966) indicates that none of the methods stochastically dominates one other approach for normal samples. The mean value of the calculated scores is set for each example to zero.

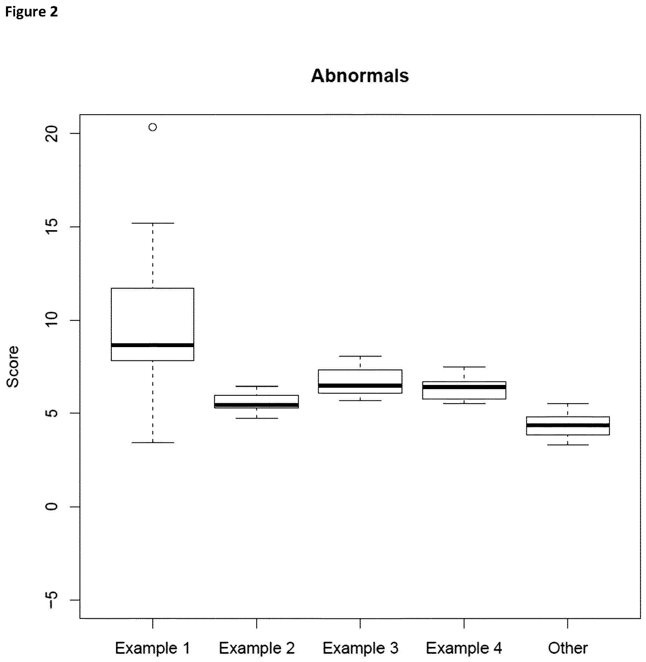

Figure 2: The Figure illustrates the score values and their respective distribution obtained by the method of the present invention in Examples 1-4 and with the state-of-the-art method (hereby termed as "other" method), for samples comprising cell-free tumor ("abnormal") DNA (said samples not included in the training step). When these scores are compared to the scores obtained from normal samples (Figure 1) the highest differentiation is achieved by the methods according to the present invention from Examples 1-4 clearly illustrating the improvement (increase) in sensitivity of the present method (Examples 1-4) over the state-of-the-art method in differentiating abnormal samples from normal samples.

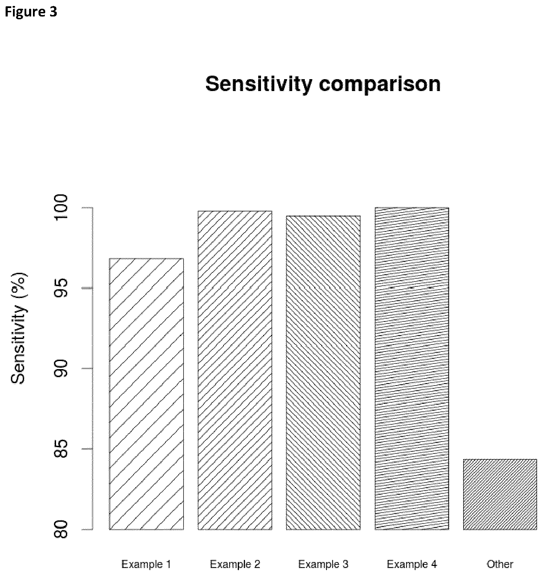

Figure 3: The figure illustrates the comparison of sensitivity performance between the methods described in Examples 1-4 and the state-of-the-art method (hereby termed as "other" method). From the empirical distributions of each of the scores of normal and abnormal samples, the estimated sensitivity was computed for all methods in Examples 1-4 and the state-of-the-art ("other") method. The specificity for all methods (i.e. significance level in statistical hypothesis testing) is set at 99.9 % with the estimated sensitivities for this dataset being equal to 96.8 %, 99.94 %, 99.48 %, 99.9997 % for the methods of examples 1-4, respectively. All methods of the present invention significantly outperform the state-of-the-art method that only achieves a sensitivity of 84.3 % as well as other methods currently available in the literature using fragment size and copy number change information to classify samples into clinically informative categories and achieve sensitivities ranging from only 60 % to 90 % (Mouliere et al. 2018 and Adalsteinsson et al. 2017) (data not shown).

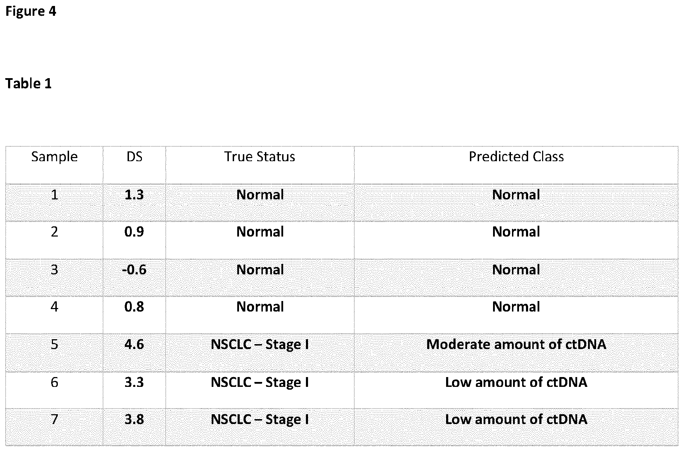

Figure 4: Table 1: The table illustrates the scores obtained by the method of the present invention in Example 4, for four additional normal samples and three additional abnormal samples, the abnormal samples being from cancer patients diagnosed with NSCLC (Stage I). The table highlights the classification of the amount of ctDNA into low, moderate and high. The amount of ctDNA in the sample is classified as low if the combined diagnostic score value is between 2 and 4.5, as moderate if the combined diagnostic score value is between 4.5 and 6 and as high if the combined diagnostic score value is more than 6.

## DETAILED DESCRIPTION OF THE INVENTION

The current invention describes a liquid biopsy method which utilizes novel bioinformatic analysis based on an expanded range of information extractable from ctDNA sequencing, and implements novel multiparameter strategies to establishing a robust, sensitive and specific liquid biopsy assay for the classification of samples into clinically relevant categories.

One embodiment of the present invention relates to a method of classifying a sample as comprising cell-free tumor DNA, said method comprising the determination of the sequence coordinates of the ends or "start and/or stop", and optionally of the start and/or stop plus and/or minus 1 base pair, of a plurality of cfDNA fragments comprised in a sample. The "start and/or stop" of a cfDNA fragment herein relates to the ends, the boundaries or the outermost base pairs or nucleotides of a cfDNA fragment. The determination of the sequence coordinates of cfDNA fragments can be accomplished by alignment to a reference sequence, wherein the reference sequence may be a DNA sequence of an organism, preferably a human DNA sequence, such as the hg19 or hg38 human genome sequence or the genome sequence of a human subject, which may be, in one embodiment, a healthy or cancer-free human subject.

In one embodiment of the invention the determination of the sequence coordinates may comprise the analysis and/or determination of the nucleic acid sequence of a plurality of cfDNA fragments, for example by sequencing analysis. In one embodiment, the determination of the sequence coordinates may further comprise the extraction or purification of nucleic acids and/or specifically cfDNA fragments from a sample, and/or the enrichment of cfDNA fragments from the sample and/or the preparation of a sequencing library from the isolated DNA, RNA or cfDNA before the sequencing analysis.

The analysis of the sequencing data may comprise the alignment of the obtained cfDNA nucleic acid sequence information to a reference genome sequence. This alignment allows for the mapping of the sequence coordinates of "start and/or stop" or ends of the analyzed cfDNA fragments to the reference genome sequence. In a preferred embodiment of the present invention, in addition to the start and/or stop coordinates of a sequenced cfDNA fragment, also the sequence coordinates of the +1 bp and -1 bp positions from the start and/or stop are determined from the reference genome sequence.

Subsequently, the frequency of each determined start and/or stop sequence coordinate in the plurality of cfDNA fragments comprised within a sample can be determined. Coordinates detected for the same cfDNA fragment (technical duplicate) or for two different cfDNA fragments (biological duplicates) are all considered in the calculation of the frequency (abundance) of each start and/or stop sequence coordinate detected in the plurality of cfDNA fragments. In a preferred embodiment of the present invention, in addition to the frequencies of each start and/or stop coordinate, also the frequency of each sequence coordinate +1 bp and -1 bp from the start and/or stop coordinates is determined within the plurality of cfDNA fragments in a sample.

In one embodiment of the present invention the ratio of the frequency of each determined reference genome coordinate over a corresponding reference frequency is determined. In a preferred embodiment this ratio of the coordinate's frequency in a sample versus a reference frequency is also calculated for each frequency of the start and/or stop +1 bp and -1 bp sequence coordinates.

Subsequently, a diagnostic score may be calculated from all frequency-ratios according to a method of the present invention, said diagnostic score being defined as the weighted sum of all frequency ratios obtained as described in Example 1, wherein the analyzed sample is classified as comprising tumor cfDNA, if the diagnostic score value is higher than the mean of a reference score by at least one standard deviation of the reference score, wherein the reference score is calculated from one or more reference values.

In one embodiment of the present invention after the determination of the start and/or stop coordinates of a plurality of cfDNA fragments comprised in a sample, all nucleic acid motifs in a reference sequence, comprised of e.g. trinucleotides (three consecutive nucleotides), tetranucleotides (four consecutive nucleotides) and/or pentanucleotides (five consecutive nucleotides), within a specific range of base pairs inwards from, but adjacent by 1 or more bp to each start and/or stop sequence coordinate, may be determined. In one embodiment of the present invention the specific range of base pairs inwards from, but adjacent by 1 or more bp to each start and/or stop sequence coordinate may be from 1 bp to 5 bp, 2 bp to 6 bp, 3 bp to 7 bp, 4 bp to 8 bp, 5bp to 9 bp, or 6 bp to 10 bp. In a preferred embodiment the range may be from 1 bp to 5 bp inwards from each start and/or stop sequence coordinate determined in the plurality of cfDNA fragments in a sample. Motifs are taken from the reference genome sequence in order to avoid inter-individual variabilities (i.e. single nucleotide polymorphisms).

Nucleic acid motifs may be determined based on each detected start and/or stop position in the reference sequence to which a cfDNA fragment was aligned to and not the actual sequence of the fragment.

Subsequently, the frequency (abundance) of each detected nucleic acid motif in the plurality of cfDNA fragments within a sample may be determined. Motifs detected for the same cfDNA fragment or for two different cfDNA fragments are all considered in the calculation of the frequency (abundance) of each motif detected in the plurality of cfDNA fragments. Following this, the ratio of each of the nucleic acid motif frequencies within the plurality of cfDNA fragments and a corresponding reference frequency is calculated. Subsequently, a diagnostic score is calculated from all frequency-ratios according to a method of the present invention, said diagnostic score being defined as the weighted sum of all frequency ratios as described in Example 2, wherein the analyzed sample is classified as comprising tumor cfDNA, if the diagnostic score value is higher than the mean of the reference score by at least one standard deviation of the reference score, wherein the reference score is calculated from one or more reference values.

In one embodiment of the present invention, following the determination of the start and/or stop coordinates of a plurality of cfDNA fragments comprised within a sample, all nucleic acid motifs in a reference sequence comprised of e.g. trinucleotides (three consecutive nucleotides), tetranucleotides (four consecutive nucleotides) and/or pentanucleotides (five consecutive nucleotides), within a specific range of base pairs outwards from, but adjacent by 1 or more bp to each start and/or stop sequence coordinate, may be determined.

In one embodiment of the present invention the specific range of base pairs outwards but adjacent by 1 or more bp to each start and/or stop sequence coordinate may be from 1 bp to 5 bp, 2 bp to 6 bp, 3 bp to 7 bp, 4 bp to 8 bp, 5bp to 9 bp, or 6 bp to 10 bp. In a preferred embodiment the range may be from 1 bp to 5 bp outwards from each start and/or stop sequence coordinate determined in the plurality of cfDNA fragments in a sample. Nucleic acid motifs may be determined based on each detected start and/or stop position in the reference sequence to which a cfDNA fragment was aligned to. Such nucleic acid motifs may comprise only the nucleic acid sequence of the reference sequence adjacent by 1 or more bp to where the cfDNA fragment aligns. Such motifs do not comprise the nucleic acid sequence of a cfDNA fragment, but comprise the sequence starting immediately outside of the start or stop coordinate in the reference sequence, e.g. start coordinate 1 bp to 5 bp outwards but adjacent to the start and/or stop.

Subsequently, the frequency of each detected nucleic acid motif in the plurality of cfDNA fragments within a sample may be determined. Motifs detected for the same cfDNA fragment or for two different cfDNA fragments are all considered in the calculation of the frequency (abundance) of each motif detected in the plurality of cfDNA fragments. Following this, the ratio of each of the nucleic acid motif frequencies within the plurality of cfDNA fragments and a corresponding reference frequency may be calculated. Finally, a diagnostic score may be calculated from all frequency-ratios according to a method of the present invention, said diagnostic score being defined as the weighted sum of all frequency ratios as described in Example 3, wherein the analyzed sample is classified as comprising tumor cfDNA, if the diagnostic score value is higher than the mean of the reference score by at least one standard deviation of the reference score, wherein the reference score is calculated from one or more reference values.

In one embodiment of the invention all of the herein afore described method steps of calculating a score from the ratios of: (a) the frequencies of the start and/or stop sequence coordinates (optionally -1 bp and/or +1 bp), (b) the frequencies of all nucleic acid motifs located inwards but adjacent by one or more bp to the start and/or stop coordinates of cfDNA fragments and (c) the frequencies of all nucleic acid motifs located outwards but adjacent by 1 or more bp to the cfDNA fragment start and/or stop coordinates, without comprising the cfDNA sequence; in comparison to reference frequencies, may be conducted in parallel or in a specific order, wherein subsequently the diagnostic score values of two or all of the steps (a), (b) and (c) may be used to calculate a combined diagnostic score value according to the methods of the present invention, as described in Example 4. According to this combined diagnostic score value, the analyzed sample is classified as comprising tumor cfDNA or circulating tumor DNA (ctDNA), if the combined diagnostic score value is higher than the mean of the reference score by at least one standard deviation of the reference score, wherein the reference score is calculated from one or more reference values.

In one embodiment, by comparing the combined diagnostic score value obtained for each abnormal sample to the reference score, the amount of tumor cfDNA or ctDNA in the sample can be classified as (a) low if the combined diagnostic score is between 2 and 4 standard deviations of the reference score, as (b) moderate if the combined score is between 4 and 6.5 standard deviations of the reference score and as (c) high if the combined score is more than 6.5 standard deviations of the reference score. (Table 1).

Cell-free nucleic acids

Herein, the mixture of nucleic acid fragments is preferably isolated from a sample taken from a eukaryotic organism, preferably a primate, more preferably a human. The sample may comprise cells or nucleic acids from different tissue types. As such, a sample may comprise intrinsically a mixture of nucleic acid fragments.

Herein "nucleic acid" or "nucleic acid sequence" may be used interchangeably with, without being limited to, DNA, RNA, genomic DNA, cell-free DNA and/or RNA, and tRNA, messenger RNA (mRNA), synthetic DNA or RNA.

In the context of the present invention, the term "nucleic acid fragments" and "fragmented nucleic acids" can be used interchangeably. In a preferred embodiment of the method according to the invention, the nucleic acid fragments are circulating cell-free DNA or RNA.

In one embodiment of the present invention a minimum of 100,000 cfDNA fragments comprised within a sample may be analyzed. In another embodiment the number of cfDNA fragments comprised within the sample to be analyzed may range from 100 thousand to 500 thousand, 500 thousand to 1 million, 1 million to 2 million, 2 million to 5 million, 5 million to 10 million, 10 million to 20 million, 20 million to 50 million or from 50 million to 500 million.

In one embodiment of the invention, a "sample" is a blood sample, a serum sample, a plasma sample, a liquid biopsy sample or a DNA sample (e.g. mixture of nucleic acid fragments) comprising cell-free DNA (cfDNA), cell-free tumor DNA (cftDNA), circulating tumor DNA (ctDNA) or circulating cftDNA. In the context of the present invention the terms "cfDNA", "cftDNA", "ctDNA" or "circulating cftDNA" may be used interchangeably.

In one embodiment, the sample is selected from the group consisting of a plasma sample, a blood sample, a urine sample, a sputum sample, a cerebrospinal fluid sample, an ascites sample and a pleural fluid sample from a subject having or suspected of having a tumor. In one embodiment, the sample or DNA sample is from a tissue sample from a subject having or suspected of having a tumor or a set of malignant cells.

In the context of the present invention the terms "tumor", "cancer" or "abnormal" may be used interchangeably. Herein the terms "cancer" or "tumor" may also comprise of early stage of cancer or advanced cancer, metastasis or precancerous tissues or cells. Herein a tumor sample or abnormal sample may relate to a sample comprising (cell-free) DNA or RNA originating from a primary tumor or a metastatic tumor. A normal sample or reference sample may herein relate to a sample comprising only (cell-free) DNA or RNA originating from non-cancerous, healthy or "normal" tissue(s) or cell(s). In the context of the present invention the terms "normal", "control" or "reference" may be used interchangeably.

The methods of the invention can be used with a variety of biological samples. Essentially any biological sample containing genetic material, e.g. RNA or DNA, and in particular cell-free DNA (cfDNA) or cell-free RNA, can be used as a sample in the methods allowing for genetic analysis of the RNA or DNA therein. For example, in one embodiment, the DNA sample is a plasma sample or a blood sample containing cell-free DNA (cfDNA).

In yet another embodiment for oncology purposes, the sample is a biological sample obtained from a subject having or suspected of having a tumor or cancer. In one embodiment, the sample comprises circulating cell-free tumor DNA (cftDNA). In another embodiment the sample is a subject's urine, sputum, ascites, cerebrospinal fluid or pleural effusion. In another embodiment, the oncological sample is a subject plasma sample, prepared from subject peripheral blood. Thus, the sample can be a liquid biopsy sample that is obtained non-invasively from a subject's blood sample, thereby potentially allowing for early detection of cancer prior to the development of a detectable or palpable tumor, or allowing monitoring of disease progression, disease treatment, or disease relapse.

Herein cell free DNA (cfDNA) refers to DNA that is not contained within a cell. A sample may comprise cfDNAs from normal or healthy cells and/or from cancer cells. Cell-free DNA may be released into the blood or serum through secretion, apoptosis or necrosis. If cfDNA is released from a tumor or cancer cell, it may be called cell-free tumor DNA (cftDNA).

In the context of the present invention, the term "subject" refers to animals, preferably mammals, and more preferably to humans or human patients. As used herein, the term "subject" may refer to a subject suffering from or suspected of having a tumor.

A "tumor" herein refers to cancer in general, including but not limited to a solid tumor, an adenoma, blood cancer, liver cancer, lung cancer, pancreatic cancer, prostate cancer, breast cancer, gastric cancer, glioblastoma, colorectal cancer, head and neck cancer, a tumor of an advanced stage of cancer, a benign or malignant tumor, a metastasis or a precancerous tissue.

Herein the "ends" of cfDNA fragments define the outermost nucleotides on the 3' and 5' ends of the nucleic acid fragment and may herein also be referred to as "start and/or stop (positions)" or "break points" or "boundaries" of a cfDNA fragment. When aligned to a reference sequence the "(start and/or stop) coordinates" or "sequence coordinates" of the cfDNA fragment are defined by the outermost nucleic acid sequence positions to which the ends of the cfDNA fragments align to in the reference sequence. For example, if a cfDNA fragment is complementary to or aligns to the reference nucleic acid sequence spanning from the sequence position 1500 bp to 1700 bp, the sequence coordinates would be 1500 and 1700 bp, defining a length of 200 bp of the cfDNA fragment.

The size profile of cfDNA exhibiting a 166-bp major peak and smaller peaks with 10-bp intervals suggested that the biology of cfDNA might be associated with nucleosomal organization. Similar patterns were also observed in plasma DNA in patients with cancer. The non-random fragmentation patterns of cfDNA, related to the tissues of origin, could also be related to the patient's health status. Hence, the ends or start and/or stop coordinates and frequency of cell-free DNA fragments are indicative of the disease progression. They vary according to the origin of the tumor and the tumor mass, which reflects the extent of the disease and hence its response to a given therapy.

As used herein the term "inwards" from a start and/or stop" coordinate refers to the direction from a "start and/or stop" coordinate of a nucleic acid fragment in a reference sequence, in which a sequence or motif extends. "Inwards" may relate to the nucleic acid sequence or motif comprised in the sequence of the nucleic acid fragment or the reference sequence it aligns to. "Inwards" might refer to be + 1, + 2, + 3, +4, +5, etc. base pairs from the start coordinate and/or - 1, - 2, - 3, - 4, - 5 base pairs from a stop coordinate of a nucleic acid fragment. In one embodiment the range of base pairs inwards but adjacent to each start and/or stop sequence coordinate can be from 1 bp to 5 bp, 2 bp to 6 bp, or 3 bp to 7 bp, or 4 bp to 8 bp, or 5 bp to 9 bp or 6 bp to 10 bp from each start and/or stop coordinate.

As used herein the term "outwards" from a start and/or stop" coordinate refers to the direction from a "start and/or stop" coordinate of a nucleic acid fragment in a reference sequence, in which a sequence extends. "Outwards" may relate to a nucleic acid sequence or motif not comprised in the sequence of the nucleic acid fragment or the reference sequence it aligns to. "Outwards" might refer to be + 1, + 2, + 3, +4, +5, etc. base pairs from the stop coordinate and/or -1, - 2, - 3, - 4, - 5 base pairs from a start coordinate of a nucleic acid fragment. In one embodiment the range of base pairs outwards but adjacent to each start and/or stop sequence coordinate can be from 1 bp to 5 bp, 2 bp to 6 bp, or 3 bp to 7 bp, or 4 bp to 8 bp, or 5 bp to 9 bp or 6 bp to 10 bp from each start and/or stop coordinate.

The present method analyzes the frequency and/or sequence motifs of the start and/or stop coordinates plus and minus 1 bp as the observed end sites of fragments might not necessarily be the true cutting/digestion sites (Peiyong Jiang et al., Genome Res., 2020, doi: 10.1101/gr.261396.120). Hence, by taking into account the likelihood of the nearby genomic bases to be the true digestion site, the present invention results in an improved accuracy over current state of the art, in the classification of biological samples into clinically relevant categories.

Herein a "nucleic acid motif", "sequence motif" or "motif" refers to an array of consecutive nucleotides in a nucleic acid sequence, comprised of 2, 3, 4, 5, 6, 7, 8, 9, 10, 20, 30, 40, 50, 100 etc. consecutive nucleotides. This array of consecutive nucleotides might also be called "trinucleotides", "tetranucleotides", "pentanucleotides", "hexanucleotides" etc. Said motifs are a subset of human genomic locations preferentially cleaved, e.g. by specific nucleases, when cell-free and/or circulating DNA molecules are generated and released into the blood plasma. Such plasma DNA end motifs resulting from nucleases that cleave nucleic acids, such as DNA, during apoptosis, display a distinct signature, which can comprise or be specific for HSNRF. In preferred embodiment, "motif" refers to an array of 3, 4 or 5 consecutive nucleotides from a reference genome sequence.

In one embodiment a nucleic acid motif might be located at the end or the break point of a cfDNA fragment, wherein the motif might be comprised within the nucleic acid sequence of the cfDNA fragment or lie outside of the boundaries of the cfDNA fragment sequence and within the reference nucleic acid sequence, for example adjacent to where the cfDNA fragment aligns.

Analysis of cfDNA

Herein a "reference sequence" may be any nucleic acid sequence, a genomic sequence, the genomic sequence of an organism or subject, preferably a sequence of the human genome (e.g. hg19 or hg38) or of a healthy individual or subject.

Herein a "reference frequency" for the frequency of a start and/or stop sequence coordinate may be the frequency of the corresponding start and/or stop sequence coordinate in one or more reference genomes, reference sequences, or in one or more genomes or sequences of one or more healthy or "normal" control samples, subjects or patients. Herein a "reference frequency" for a nucleic acid motif may be the frequency of the corresponding nucleic acid motif in one or more reference genomes, reference sequences, or in one or more genomes or sequences of one or more healthy or "normal" control samples, subjects or patients.

Herein a "frequency" may be used interchangeably with abundance and occurrence. In one embodiment of the invention a "frequency" describes the abundance and occurrence or the number of, for example, nucleic acid sequence motifs, nucleic acid (cfDNA) fragments or start and/or stop sequence coordinates that were detected or counted in a plurality of nucleic acids or cfDNA fragments comprised in a sample.

Herein a "ratio" may refer to the mathematical relation or proportion of the frequency of, for example, a nucleic acid sequence motif detected in a plurality of nucleic acid fragments in a sample to the frequency of the same nucleic acid sequence motif in a reference sample. Herein a ratio may be calculated by dividing the frequency of each coordinate or motif over a corresponding reference frequency of a corresponding coordinate or motif.

For the sample preparation, nucleic acids, such as DNA and/or RNA, are extracted from the sample using standard techniques known in the art, a non-limiting example of which is the QIAsymphony (QIAGEN) protocol, QIAamp Circulating Nucleic Acid (QIAGEN), KingFisher (Thermofisher) protocol, MagMAX™ Cell-Free DNA (Thermofisher) or any other manual or automated extraction method suitable for cell free DNA isolation.

Following isolation, the cell-free DNA of the sample may be used for sequencing library preparation to make the sample compatible with a downstream sequencing technology, such as Next Generation Sequencing (NGS). Typically, this involves ligation of adapters onto the ends of the cell-free DNA fragments. Sequencing library preparation kits are commercially available or can be developed.

Targeted enrichment of cfDNA is performed using Target Capture Sequences (TACS) which bind to regions of interest on the human genome and wherein: each sequence within the pool is between 125-260 base pairs in length and/or 125-300 bp in length, and/or 125-350 bp in length, each sequence having a 5' end and a 3' end; each sequence within the pool binds to the region of interest at least 10 base pairs away, on both the 5' end and the 3' end, from regions harboring Copy Number Variations, Segmental duplications or repetitive DNA elements; and the GC content of the TACS is between 20%-50%, and/or 20%-60%, and/or 20%-70% and/or 20%-80%.

Herein the term "Target Capture Sequences" or "TACS" refers to DNA sequences that are complementary to the region(s) of interest on a genomic sequence(s) of interest and which are used as "bait" to capture and enrich the region of interest from a large library of sequences, such as whole genomic sequencing library prepared from a biological sample. In the context of the present invention the terms "Target Capture Sequences" or "TACS" or "probes" may be used interchangeably.

In another embodiment, the pool of TACS binds to a plurality of tumor biomarker sequences of interest selected from a group comprising but not limited to, AKT1, ALK, APC, AR, ARAF, ATM, BAP1, BARD1, BMPR1A, BRAF, BRCA1, BRCA2, BRIP1, CDH1, CDK4, CDKN2A (pl4ARF), CDKN2A (pl6INK4a), CHEK2, CTNNB1, DDB2, DDR2, DICERI, EGFR, EPCAM, ERBB2, ERBB3, ERBB4, ERCC1, ERCC2, ERCC3, ERCC4, ERCC5, ESR1, FANCA, FANCB, FANCC, FANCD2, FANCE, FANCF, FANCG, FANCI, FANCL, FANCM, FBXW7, FGFR1, FGFR2, FLT3, FOXA1, FOXL2, GATA3, GNA11, GNAQ, GNAS, GREM1, HOXB13, IDH1, IDH2, JAK2, KEAP1, KIT, KRAS, MAP2K1, MAP3K1, MEN1, MET, MLH1, MPL, MRE11A, MSH2, MSH6, MTOR, MUTYH, MYC, MYCN, NBN, NPM1, NRAS, NTRK1, PALB2, PDGFRA, PIK3CA, PIK3CB, PMS2, POLD1, POLE, POLH, PTEN, RAD50, RAD51C, RAD51D, RAF1, RBI, RET, ROS1, RUNX1, SDHA, SDHAF2, SDHB, SDHC, SDHD, SLX4, SMAD4, SMARCA4, SPOP, STAT, STK11, TMPRSS2, TP53, VHL, XPA, XPC and combinations thereof. In one embodiment, the pool of TACS binds to a plurality of tumor biomarker sequences of interest selected from a group comprising EGFR_6240, KRAS_521, EGFR_6225, NRAS_578, NRAS_580, PIK3CA_763, EGFR_13553, EGFR_18430, BRAF_476, KIT_1314, NRAS_584, EGFR_12378, and combinations thereof.

In another embodiment, the pool of TACS binds to a plurality of tumor biomarker sequences of interest selected from a group comprising but not limited to COSM6240 (EGFR_6240), COSM521 (KRAS_521), COSM6225 (EGFR_6225), COSM578 (NRAS_578), COSM580 (NRAS_580), COSM763 (PIK3CA_763), COSM13553 (EGFR_13553), COSM18430 (EGFR_18430), COSM476 (BRAF_476), COSM1314 (KIT_1314), COSM584 (NRAS_584), COSM12378 (EGFR_12378), and combinations thereof, wherein the identifiers refer to the COSMIC database ID number of the biomarker. In general, a probe-hybridization or enrichment step, can be carried out before the sequencing library is created or after the library has been created.

In one embodiment of the present invention, the sequencing library might be enriched for sequence regions of interest by hybridization of the library to one or more probes covering e.g. hot spots of non-random fragmentation (HSNRF). Such HSNFR regions are regions with high probability of comprising, within a short distance, numerous nucleic acid sequence variations facilitating the identification of different tissue types of origin (e.g. cancer and normal), which are present in a mixture of cfDNA.

The region(s) of interest on the chromosome(s) of interest where the HSNRF lie are enriched by hybridizing the pool of HSNRF-capture probes to the sequencing library, followed by isolation of those sequences within the sequencing library that bind to the probes. In one embodiment, the probe spans a HSNRF site such that only the 5' end of the fragmented cell-free nucleic acids is captured by the probe. In another embodiment the probe spans a HSNRF site such that only the 3' end of the fragmented cell-free nucleic acids arising from HSNRF can bind to the probe. In another preferred embodiment, the probe spans both HSNRF sites associated with a fragmented nucleic acid such that both the 5' and the 3' end of a cell-free nucleic acid associated with the given HSNRF site are captured by the probe.

To facilitate isolation of the desired, enriched sequences (HSNRF), typically the probe sequences are modified in such a way that sequences that hybridize to the probes can be separated from sequences that do not hybridize to the probes. Typically, this is achieved by fixing the probes to a support. This allows for physical separation of those sequences that bind the probes from those sequences that do not bind the probes. For example, each sequence within the pool of probes can be labeled with biotin and the pool can then be bound to beads coated with a biotin-binding substance, such as streptavidin or avidin. In a preferred embodiment, the probes are labeled with biotin and bound to streptavidin-coated magnetic beads, thereby allowing separation by exploiting the magnetic property of the beads. The ordinarily skilled artisan will appreciate, however, that other affinity binding systems are known in the art and can be used instead of biotin-streptavidin/avidin. For example, an antibody-based system can be used in which the probes are labeled with an antigen and then bound to antibody-coated beads. Moreover, the probes can incorporate on one end a sequence tag and can be bound to a support via a complementary sequence on the support that hybridizes to the sequence tag. Furthermore, in addition to magnetic beads, other types of supports can be used, such as polymer beads, glass and the like.

In certain embodiments, the members of the sequencing library that bind to the pool of probes are fully complementary to the probe. In other embodiments, the members of the sequencing library that bind to the pool of probes are partially complementary to the probe. For example, in certain circumstances it may be desirable to utilize and analyze data that are from DNA fragments that are products of the enrichment process but do not necessarily belong to the genomic regions of interest (i.e. such DNA fragments could bind to the probe because of partial homologies) and when sequenced would produce very low coverage throughout the genome across non-probe coordinates.

Following enrichment of the sequence(s) of interest using the probes, thereby forming an enriched library of DNAs with HSNRF sites, the members of the enriched HSNRF library are eluted and are amplified and sequenced using standard methods known in the art. In another embodiment, the probes are provided together with a support, such as biotinylated probes provided together with streptavidin-coated magnetic beads.

For detection of tumor biomarkers, probes are designed based on the design criteria described herein and the known sequences of tumor biomarker genes and genetic mutations therein associated with cancer. In one embodiment, a plurality of probes used in the method bind to a plurality of tumor biomarker sequences of interest. Here, the probe may lie in the hot spots of non-random fragmentation adjacent to the mutation site.

Herein, Next Generation Sequencing (NGS) may be used for nucleic acid sequence analysis, although other sequencing technologies can also be employed, which provide very accurate counting in addition to sequence information. Accordingly, other accurate counting methods, such as but not limited to digital PCR, single molecule sequencing, nanopore sequencing, DNA nanoball sequencing, sequencing by ligation, Ion semiconductor sequencing, sequencing by synthesis, and microarrays can also be used instead of NGS.

In one embodiment the invention relates to a method, wherein the nucleic acid fragments to be detected or the origin of which is to be determined, are present in the mixture at a concentration lower than a nucleic acid fragment from the same genetic locus but of different origin.

The present method is particularly suited to analyze such low concentrations of target cfDNA. In the method according to the invention, the nucleic acid fragment to be detected or the origin of which is to be determined and the nucleic acid fragment from the same genetic locus but of different origin are present in the mixture at a ratio selected from the group of 1:2, 1:4, 1:10, 1:20, 1:50, 1:100, 1:200, 1:500, 1:1000, 1:2000 and 1:5000. The ratios are to be understood as approximate ratios which means plus/minus 30%, 20% or 10%. A person skilled in the art knows that such ratios will not occur at exactly the numerical values cited above. The ratios refer to the number of locus-specific molecules for the rare type to the number of locus-specific molecules for the abundant type.

Data analysis

The information obtained from sequencing of the enriched library is analyzed using an innovative biomathematical/biostatistical data analysis pipeline. The present method makes use of features of cfDNA fragments including the combination of all possible motifs adjacent by 1 or more bp to the end coordinates using a reference genome sequence and excluding the observed cfDNA end sites since they might not represent the true digestion sites. Furthermore, by combining the analysis of different features of cfDNA, including locations and motifs, the current invention achieved an unexpected technical effect of improved accuracy, i.e increased sensitivity at the same specificity levels.

According to a preferred embodiment of the invention, targeted paired-end next generation sequencing is performed. The multiplexed data for all samples are demultiplexed using Illumina bcltofastq tool. Said sample's sequencing data are processed to remove adaptor sequences and poor-quality reads (Q-score <25) using the cutadapt software (Martin, M. et al. 2011 EMB.net-Journal 17.1).

Processed reads, which were at least 25 bases long, were aligned to the human reference genome build GRCh37 (hg19) (UCSC Genome Bioinformatics) using the Burrows-Wheel Alignment algorithm (Li, H. and Durbin, R. (2009) Bioinformatics 25:1754-1760). Paired-reads with insert size greater than a threshold value were removed, said threshold value being in the range 100-600. If relevant, duplicate reads are identified, grouped by Unique Molecular Identifier (UMI) family and used to produce consensus reads per UMI family, post-alignment.

Where applicable, sequencing output pertaining to the same sample but processed on separate sequencing lanes, was merged to a single sequencing output file. The utilization of duplicates and merging procedures were performed using fgbio, picard tools software suites (Broad Institute) and the Sambamba tools software suite (Sambamba reference, Tarasov, Artem, et al. Sambamba: fast processing of NGS alignment formats. Bioinformatics 31.12 (2015): 2032-2034). Information in terms of mapping positions (outermost and nearby coordinates), read-depth per base at loci of interest, and fragment size was obtained using the mpileup option of the SAMtools software suite, from here on referred to as the mpileup file, and processed using custom-build application programming interfaces (APIs) written in the Python and R programming languages (Python Software Foundation (2015) Python; The R Foundation (2015) The R Project for Statistical Computing).

An end coordinate of a fragment is defined as the outermost coordinate in the reference genome which is spanned by the fragment, i.e. each aligned fragment has two end coordinates (a start/leftmost position (5' end) and a stop/right-most position (3' end) coordinate relative to the reference genome).

In various embodiments of the invention, the targeted panel consisted of a minimum of 500 targeted genomic bases. The minimum number of fragments needed per sample is 100,000.

Herein a "diagnostic score value" is calculated as the weighted sum of all frequency ratios as described in Examples 1, 2 and 3 in the 'Examples section".

Herein a "combined diagnostic score value" is calculated as the weighted sum of at least two or more frequency ratios from all steps described in the current invention, as described in Example 4.

In one embodiment of the present invention a "reference score" may be calculated from one or more "reference values".

In one embodiment a reference value or reference score may be calculated from data acquired from one or more normal or reference samples. In one embodiment the reference value or the reference score, and the value of the analyzed sample (e.g. the frequencies of nucleic acid motifs or the frequencies of start and/or stop coordinates) or the diagnostic score for the analyzed sample it is compared to, are calculated according to the same calculation method, as disclosed herein.

Sample classification

Herein the classification of a sample comprises binary classification (i.e. cancer, no cancer; good prognosis, bad/poor prognosis; relapsing, non-relapsing) and classification of the amount of the cftDNA into low, moderate and high amounts.

Clinically relevant categories for classification of a sample may be the presence or absence of cancer, disease or cancer remission, relapsing of the disease or cancer, early cancer stages and prognosis.

In one embodiment, the amount, presence or abundance of tumor cfDNA in the sample can be classified as low if the combined diagnostic score is between 2 and 4 standard deviations of the reference scores, as moderate if the combined score is between 4 and 6.5 standard deviations of the reference scores and high if the combined score is more than 6.5 standard deviations of the reference scores.

Oncology Uses

The present invention may be used in the treatment of cancer or for assessing tumor burden, detecting minimal residual disease, monitoring treatment outcome, long term monitoring of patient outcome. The present invention may be further used in the identification of mutations suitable for targeted therapy and in the detection of cancer somatic and germline mutations. The present method facilitates early detection of small tumors that are not detectable by other methods and enables a more targeted, customized treatment approach.

Kits

In another aspect, the invention provides kits for performing the method of the invention. In one embodiment, the kit comprises a container consisting of the pool of probes, and software and instructions for performing the method.

In addition to the pool of probes, the kit can comprise one or more of the following (i) one or more components for isolating cell-free DNA from a biological sample, (ii) one or more components for preparing and enriching the sequencing library (e.g., primers, adapters, buffers, linkers, DNA modifying enzymes, ligation enzymes, polymerase enzymes, probes and the like), (iii) one or more components for amplifying and/or sequencing the enriched library, and/or (iv) software for performing statistical analysis. Components suitable for carrying out the steps referred to in (i), (ii) and (iii) are well known to the person skilled in the art.

In one embodiment, the probes are provided in a form that allows them to be bound to a solid support, such as biotinylated probes. In another embodiment, the probes are provided together with a solid support, such as biotinylated probes provided together with streptavidin-coated magnetic beads.

In various other embodiments, the kit can comprise additional components for carrying out other aspects of the method. For example, in addition to the pool of probes, the kit can comprise one or more of the following (i) one or more components for isolating cell free DNA from a maternal plasma sample; (ii) one or more components for preparing the sequencing library (e.g., primers, adapters, linkers, restriction enzymes, ligation enzymes, polymerase enzymes); (iii) one or more components for amplifying and/or sequencing the enriched library; and/or (iv) software for performing statistical analysis. Components suitable for carrying out the steps referred to in (i), (ii) and (iii) are well known to the person skilled in the art.

## EXAMPLES

### Example 1

The determination of the start and/or stop (plus and/or minus 1 base pair) of a plurality of cfDNA fragments comprised in a sample was accomplished by alignment to a reference sequence. Subsequently, the frequency of each determined start and/or stop sequence coordinate in the plurality of cfDNA fragments comprised within a sample was determined. The ratio of the frequency of each determined reference genome coordinate over a corresponding reference frequency was determined, and the weighted sum (herein referred to as the "diagnostic score") of all frequency ratios obtained was calculated.

According to one embodiment of the present invention for each base $i$, for $i = 1, ...,B$, with $B$ being equal to the total number of targeted bases in said panel, a random variable $X_i$ was defined as the total number of mapped reads satisfying at least one of the following conditions:

> (A1) having a start position coordinate at base $i$, or
> (A2) having a stop position coordinate at base $i$, or
> (A3) having a start minus one base position coordinate at base $i$, or
> (A4) having a start plus one base position coordinate at base $i$, or
> (A5) having a stop minus one base position coordinate at base $i$, or
> (A6) having a stop plus one base position coordinate at base $i$.

Under the null hypothesis (i.e. background model) it is expect to observe a different but stationary number of reads satisfying at least one of the conditions A1-A6 at different bases of the genome, said per-base background probability distribution model being estimated from a set of normal samples. From the definition of $X_i$ above, we have that $X_i \sim Bin(x_i; n_i, p_i),$ with $n_i$ being equal to the total number of reads spanning base $i$ and $p_i$ being estimated for all $i$, say $\widehat{p_i}$, as follows:

$$\widehat{p_i} = \frac{\sum_{j=1}^{N} z_{i,j}}{\sum_{j=1}^{N} n_{i,j}},$$

where $z_{i,j}$ is the observed number of reads satisfying at least one of the conditions A1-A6 at base $i$ for normal sample $j$, and $n_{i,j}$ is the total number of reads spanning base $i$ for normal sample $j$ out of $N$ normal samples in total. A Binomial distribution with a very small $p$ and large $n$ can be approximated by a Poisson distribution with rate parameter equal to $np$. Hence, the per-base background model is defined by the following mathematical formula:

$$X_i \sim Po(\widehat{p_i} n_i),$$

with $n_i$ being equal to the total number of reads spanning base $i$. In another embodiment of the invention, a Weibull or Beta distribution is used to model, at each base $i$, the random variable defined by $z_{i,j}/n_{i,j}$ for all $j$.

After training the per-base background model, it was proceeded as follows. For each sample k, in one embodiment of the invention, the following is performed: for each $X_i$ the observed value, say $x_i$, was compared against the estimated per-base background model. If the p-value, i.e. $P(X_i > x_i) = 1 - P(X_i \leq x_i)$, was less than 0.001, then the observed value of $X_i$ was divided by the total number of reads spanning base $i$, i.e. $Y_i = X_i/n_i$, otherwise $Y_i = 0$. The sample specific score is, subsequently, computed as follows: $S_{0,k} = \frac{1}{n_2} \sum_{i=1}^{n_2} Y_i,$ where $n_2$ is the total number of bases with $Y_i > 0$. Then $S_{0,k}$ is normalized to get the normalized score $S_{1,k}$ using the following mathematical formula:

$$S_{1,k} = \frac{S_{0,k} - m}{s},$$

where m and s are the mean and standard deviation of all $S_0$ values from normal reference samples. (Figures 1, 2 and 3).

### Example 2

Following the determination of the start and/or stop (plus and/or minus 1 base pair) sequence coordinates of the cfDNA fragment, all nucleic acid motifs in a reference sequence from the reference genome were determined. Said motifs comprised of trinucleotides, tetranucleotides and/or pentanucleotides and were within a specific range of base pairs inwards but adjacent by 1 or more base pairs of the start and/or stop coordinates. The ratio of the frequency of each of the nucleic acid motif frequencies within the plurality of cfDNA fragments over a corresponding reference frequency was determined, and the weighted sum (herein referred to as the "diagnostic score") of all frequency ratios obtained was calculated.

According to one embodiment of the invention, for each sample, say $k$, two sequences for each cfDNA fragment aligned on the hg19 reference genome were determined, said sequences comprising the hg19 genome sequence within a range of 1 to 5 base pairs inwards from the two ends of the aligned cfDNA fragments (excluding the nucleic acid sequence spanned by the fragment) and calculated the absolute frequency of all trinucleotide (e.g. ACC, GGT, etc.), tetranucleotide and pentanucleotide sequence motifs within said sequences, say $T_{ij}$ for $i = 1, ..., n_j$, $j = 3,4,5$ is the number of nucleotides and $n_j$ is the number of all possible $j$-nucleotide motifs ($n_3 = 64$, $n_4 = 256$, $n_5 = 1024$). The sample specific score $S_{2,k}$ is calculated as follows:

$$S_{2,k} = \sum_{j=3}^{5} S_{2,jk} b_j$$

$$S_{2,jk} = \frac{1}{n_j} \sum_{i=1}^{n_j} \chi_{ij}^2 w_{ij}$$
*where*

$$\chi_{ij}^2 = \left( \frac{\widetilde{f_{ij}} - m_{ij}}{s_{ij}} \right)^2 ,$$

$$\widetilde{f_{ij}} = \frac{f_{ij}}{r_{ij}} ,$$

$$f_{ij} = \frac{T_{ij}}{D_k}$$

In the above formulas $D_k$ is the total number of consensus fragments in sample $k$, $r_{ij}$ is the reference value of $f_{ij}$ calculated from a training data set of ctDNA-free samples, $m_{ij}$ and $s_{ij}$ are reference mean and standard deviation of

$$\widetilde{f_{ij}}$$

calculated from a training data set of ctDNA-free samples, $w_{ij}$ are weights $\left( \sum_{i=1}^{n_j} w_{ij} = 1 \right)$ that are optimized from a training set in order to provide the optimal separation between normal and abnormal samples. The weights $b_j$ can vary in various embodiments of the invention $b_3 = 1/12$ or $1/6$ or $1/3$ or $1/2$, $b_4 = 1/12$ or $1/6$ or $1/3$ or $1/2$ and $b_5 = 1 - b_3 - b_4$. (Figures 1, 2 and 3).

**Example 3**

Following the determination of the start and/or stop (plus and/or minus 1 base pair) sequence coordinates of the cfDNA fragment, all nucleic acid motifs in a reference sequence from the reference genome were determined. Said motifs comprised of trinucleotides, tetranucleotides and/or pentanucleotides and were within a specific range of base pairs outwards but adjacent by 1 or more base pairs of the start and/or stop coordinates. The ratio of the frequency of each of the nucleic acid motif frequencies within the plurality of cfDNA fragments over a corresponding reference frequency was determined, and the weighted sum (herein referred to as the "diagnostic score") of all frequency ratios obtained was calculated.

In one embodiment of the method, for each sample, say $k$, two sequences for each cfDNA fragment aligned on the hg19 reference genome were determined, said sequences comprising the hg19 genome sequence within a range of 1 to 5 base pairs outwards from the two ends of the aligned cfDNA fragments (excluding the nucleic acid sequence spanned by the fragment) and calculated the absolute frequency of all trinucleotide (e.g. ACC, GGT, etc), tetranucleotide and pentanucleotide sequence motifs within said sequences, say $T_{ij}$ for $i = 1, ..., n_j$, $j = 3,4,5$ is the number of nucleotides and $n_j$ is the number of all possible $j$-nucleotide motifs ($n_3 = 64$, $n_4 = 256$, $n_5 = 1024$). The sample specific score $S_{3,k}$ is calculated as follows:

$$S_{3,k} = \sum_{j=3}^{5} S_{3,jk} b_j$$

$$S_{3,jk} = \frac{1}{n_j} \sum_{i=1}^{n_j} \chi_{ij}^2 w_{ij}$$
*where*

$$\chi_{ij}^2 = \left( \frac{\widetilde{f_{ij}} - m_{ij}}{s_{ij}} \right)^2 ,$$

$$\widetilde{f_{ij}} = \frac{f_{ij}}{r_{ij}} ,$$

$$f_{ij} = \frac{T_{ij}}{D_k}$$

In the above formulas $D_k$ is the total number of consensus fragments in sample $k$, $r_{ij}$ is the reference value of $f_{ij}$ calculated from a training data set of ctDNA-free samples, $m_{ij}$ and $s_{ij}$ are reference mean and standard deviation of

$$\widetilde{f_{ij}}$$

calculated from a training data set of ctDNA-free samples, $w_{ij}$ are weights $\left(\sum_{i=1}^{n_j} w_{ij} = 1\right)$ that are optimized from a training set in order to provide the optimal separation between normal and abnormal samples. The weights $b_j$ can vary in various embodiments of the invention $b_3$ = 1/12 or 1/6 or 1/3 or 1/2, $b_4$ = 1/12 or 1/6 or 1/3 or 1/2 and $b_5$ = 1 - $b_3$ - $b_4$. (Figures 1, 2 and 3).

**Example 4**

In one embodiment of the method, a weighted sum of at least two of the scores calculated in examples 1, 2 and 3 was computed for each sample, said weighted sum referred to as "combined diagnostic score" in the sequel. The diagnostic score for sample $k$, say $DS_k$, is defined as the weighted average of at least two of the scores described in examples 1, 2 and 3 above, that is

$$DS_k = \sum_{i=1}^{3} w_i S_{i,k},$$

where $S_1$, $S_2$ and $S_3$ are calculated in example 1, 2 and 3, respectively, and, in various embodiments of the invention, $w_1$ = 0.5 or 0.4 or 0.3 or 0.2 or 0 in one decimal place rounding, $w_2$ = 0.5 or 0.4 or 0.3 or 0.2 or 0 in one decimal place rounding, with $w_3$ = 1 - $w_1$ - $w_2$. In another embodiment of the method a weighted average of the maximum and minimum of $\{S_1, S_2, S_3\}$ is used to calculate the $DS$ score for sample $k$, that is $DS_k$ = $z$MAX($S_{1,k}$, $S_{2,k}$, $S_{3,k}$) + (1 - $z$)MIN($S_{1,k}$, $S_{2,k}$, $S_{3,k}$), with 0.5 < $z$ < 1.

**Claims**

1. Method of classifying a sample as comprising cell-free tumor DNA, the method comprising the steps of:

(i) determining in a sample comprising a plurality of cell-free DNA (cfDNA) fragments the sequence coordinates of the start and/or stop, and of the start and/or stop plus and/or minus 1 base pair, of at least 100,000 cfDNA fragments by alignment to a reference sequence,
(ii) determining the frequency of each coordinate determined in (i) in the plurality of cfDNA fragments comprised in the sample,
(iii) calculating the ratio of the frequency of each coordinate determined in (ii) over a corresponding reference frequency,
(iv) calculating a diagnostic score from all ratios determined in (iii) said score being the weighted sum of all frequency ratios determined in (iii), and

(v) determining a classification of the sample by comparing the diagnostic score to a reference score,
wherein the sample is classified as comprising tumor cfDNA, if the diagnostic score value is higher than the mean of the reference score by at least one standard deviation of the reference score, wherein the reference score is calculated from one or more reference values.

2. Method of classifying a sample as comprising cell-free tumor DNA, the method comprising the steps of:

(i) determining in a sample comprising a plurality of cell-free DNA (cfDNA) fragments the sequence coordinates of the start and/or stop of at least 100,000 cfDNA fragments by alignment to a reference sequence,
(ii) determining in the reference sequence all nucleic acid motifs comprised of trinucleotides, tetranucleotides and pentanucleotides within the range of 1 to 5 base pairs inwards but adjacent to each start and/or stop sequence coordinate determined in (i),
(iii) determining the frequency of each of the nucleic acid motifs determined in (ii) in the plurality of cfDNA fragments comprised in the sample,
(iv) calculating the ratio of each of the frequencies determined in (iii) over a corresponding reference frequency,
(v) calculating a diagnostic score from all ratios determined in (iv) said score being the weighted sum of all frequency ratios determined in (iv), and
(vi) determining a classification of the sample by comparing the diagnostic score to a reference score,
wherein the sample is classified as comprising tumor cfDNA, if the diagnostic score value is higher than the mean of the reference score by at least one standard deviation of the reference score, wherein the reference score is calculated from one or more reference values.

3. Method of classifying a sample as comprising cell-free tumor DNA, the method comprising the steps of:

(i) determining in a sample comprising a plurality of cell-free DNA (cfDNA) fragments the sequence coordinates of the start and/or stop of at least 100,000 cfDNA fragments by alignment to a reference sequence,
(ii) determining in the reference sequence all nucleic acid motifs comprised of trinucleotides, tetranucleotides and pentanucleotides within the range of 1 to 5 base pairs outwards but adjacent to each start and/or stop sequence coordinate determined in (i),

(iii) determining the frequency of each of the nucleic acid motifs determined in (ii) in the plurality of cfDNA fragments comprised in the sample,

(iv) calculating the ratio of each of the frequencies determined in (iii) over a corresponding reference frequency,

(v) calculating a diagnostic score from all ratios determined in (iv), said score being the weighted sum of all frequency ratios determined in (iv), and

(vi) determining a classification of the sample by comparing the diagnostic score to a reference score,

wherein the sample is classified as comprising tumor cfDNA, if the diagnostic score value is higher than the mean of the reference score by at least one standard deviation of the reference score, wherein the reference score is calculated from one or more reference values.

4. Method of classifying a sample as comprising cell-free tumor DNA, the method comprising the steps of:

(i) determining in a sample comprising a plurality of cell-free DNA (cfDNA) fragments the sequence coordinates of the start and/or stop of at least 100,000 cfDNA fragments by alignment to a reference sequence,

(ii) determining in the reference sequence all nucleic acid motifs comprised of trinucleotides, tetranucleotides and pentanucleotides:

a) within the range of 1 to 5 base pairs inwards but adjacent to each start and/or stop sequence coordinate determined in (i), and/or

b) within a range of 1 to 5 base pairs outwards but adjacent to each start and/or stop sequence coordinate determined in (i),

(iii) determining the frequency of:

a) each sequence coordinate plus and/or minus 1 base pair determined in (i) in the plurality of cfDNA fragments comprised in the sample,

b) each of the nucleic acid motifs determined in (ii) a) and b) in the plurality of cfDNA fragments comprised in the sample,

(iv) calculating the ratio of each of the frequencies determined in (iii) a) and b) over a corresponding reference frequency,

(v) calculating a diagnostic score separately for each ratio determined in step (iv), said score being the respective weighted sum of all respective frequency ratios of step (iv)

(vi) calculating a combined diagnostic score from at least two or more of the diagnostic scores determined in (v) said score being the weighted sum of said two or more diagnostic scores determined in (v), and

(vii) determining a classification of the sample by comparing the combined diagnostic score to a reference score,

wherein the sample is classified as comprising tumor cfDNA, if the combined diagnostic score value is higher than the mean of the reference score by at least one standard deviation of the reference score, wherein the reference score is calculated from one or more reference values.

5. The method of claim 4, wherein the combined diagnostic score is calculated from all of the diagnostic scores calculated in claim 4 step (v).

6. The method of claims 2 to 5, wherein the range of base pairs inwards but adjacent to each start and/or stop sequence coordinate can be from 2 bp to 6 bp, or 3 bp to 7 bp, or 4 bp to 8 bp, or 5 bp to 9 bp or 6 bp to 10 bp from each start and/or stop coordinate.

7. The method according to any of the claims 1 to 6, wherein the minimum amount of cfDNA fragments comprised within a sample to be analyzed is between 100 thousand to 500 thousand, 500 thousand to 1 million, 1 million to 2 million, 2 million to 5 million, or 5 million to 10 million, or 10 million to 20 million, or 20 million to 50 million, or 50 million to 500 million.

8. The method according to claims 4 to 7, wherein the amount of tumor cfDNA in the sample can be classified as low if the combined diagnostic score is between 2 and 4 standard deviations of the reference scores, as moderate if the combined score is between 4 and 6.5 standard deviations of the reference scores and high if the combined score is more than 6.5 standard deviations of the reference scores.

9. The method according to any of the claims 1 to 8, wherein the reference samples can be samples from cancer free patients, or from non-relapsed patients, or from successfully treated cancer patients.

10. The method according to any one of claims 1 to 8, wherein step (i) comprises the determination of the nucleic acid sequence of at least a portion of the plurality of cfDNA fragments in the sample prior to the alignment to a reference sequence.

11. The method according to claim 1-10, wherein step (i) further comprises the enrichment of cfDNA fragments prior to the determination of the nucleic acid sequence of cfDNA fragments.

12. The method according to any one of the preceding claims, wherein the sample is classified as comprising tumor cfDNA originating from a tumor selected from the group of blood cancer, liver cancer, lung cancer, pancreatic cancer, prostate cancer, breast cancer, gastric cancer, glioblastoma, colorectal cancer, head and neck cancer, a solid tumor, a benign tumor, a malignant tumors, an advanced stage of cancer, a metastasis or a precancerous tissue.

13. A kit comprising:

(i) components for carrying out the method according to any of the claims 1 to 12, wherein components comprise:

a) one or more components for isolating cell-free DNA from a biological sample,
b) one or more components for preparing and enriching the sequencing library, and/or
c) one or more components for amplifying and/or sequencing the enriched library,

(ii) software for performing statistical analysis.

**Figure 1**

**Figure 2**

**Figure 3**

Sensitivity comparison

Figure 4

Table 1

| Sample | DS | True Status | Predicted Class |
|--------|------|------------------|---------------------------|
| 1 | 1.3 | Normal | Normal |
| 2 | 0.9 | Normal | Normal |
| 3 | -0.6 | Normal | Normal |
| 4 | 0.8 | Normal | Normal |
| 5 | 4.6 | NSCLC – Stage I | Moderate amount of ctDNA |
| 6 | 3.3 | NSCLC – Stage I | Low amount of ctDNA |
| 7 | 3.8 | NSCLC – Stage I | Low amount of ctDNA |

Europäisches
Patentamt

European
Patent Office

Office européen
des brevets

# EUROPEAN SEARCH REPORT

Application Number

EP 20 21 5730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PEIYONG JIANG ET AL: "Preferred end coordinates and somatic variants as signatures of circulating tumor DNA associated with hepatocellular carcinoma", PROCEEDINGS OF THE NATIONAL ACADEMY OF SCIENCES, vol. 115, no. 46, 29 October 2018 (2018-10-29), pages E10925-E10933, XP055524267, ISSN: 0027-8424, DOI: 10.1073/pnas.1814616115 * abstract * * page E10926, column 1, paragraph 2 from bottom * * page E10927, column 1, last paragraph * * page E10928, column 2, last paragraph * | 1-13 | INV. C12Q1/6881 C12Q1/6886 G16B30/00 |
| X | PEIYONG JIANG ET AL: "Preferred end coordinates and somatic variants as signatures of circulating tumor DNA associated with hepatocellular carcinoma", PROCEEDINGS OF THE NATIONAL ACADEMY OF SCIENCES, vol. 115, no. 46, 29 October 2018 (2018-10-29), pages E10925-E10933, XP055524306, ISSN: 0027-8424, DOI: 10.1073/pnas.1814616115 * Paragraph entitled: "Bioinformatics approaches to identify plasma DNA preferred ends" * | 1-13 | |
| A | WO 2018/112100 A2 (BELLWETHER BIO INC [US]) 21 June 2018 (2018-06-21) * abstract; claims 1-3 * | 1-13 | TECHNICAL FIELDS SEARCHED (IPC) C12Q G06F G16B |
| A | WO 2018/009723 A1 (GUARDANT HEALTH INC [US]) 11 January 2018 (2018-01-11) * abstract; claims 1-4 * | 1-13 | |

The present search report has been drawn up for all claims

| Place of search | Date of completion of the search | Examiner |
|---|---|---|
| Munich | 21 May 2021 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 5730

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2021

| Patent document cited in search report | | Publication date | Patent family member(s) | | Publication date |
|---|---|---|---|---|---|
| WO 2018112100 | A2 | 21-06-2018 | EP | 3555311 A2 | 23-10-2019 |
| | | | US | 2019309374 A1 | 10-10-2019 |
| | | | WO | 2018112100 A2 | 21-06-2018 |
| WO 2018009723 | A1 | 11-01-2018 | AU | 2017292854 A1 | 24-01-2019 |
| | | | BR | 112019000296 A2 | 16-04-2019 |
| | | | CA | 3030038 A1 | 11-01-2018 |
| | | | CN | 109689891 A | 26-04-2019 |
| | | | EP | 3481966 A1 | 15-05-2019 |
| | | | JP | 2019531700 A | 07-11-2019 |
| | | | KR | 20190026837 A | 13-03-2019 |
| | | | SG | 11201811556R A | 30-01-2019 |
| | | | WO | 2018009723 A1 | 11-01-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HEITZER E. et al.** *Nat. Rev. Genet.,* 2019, vol. 20 (2), 71-88
- **SMITH C.G. et al.** *Genome Med.,* 2020, vol. 12 (1), 23
- **PEIYONG JIANG et al.** *PNAS,* 2018, vol. 115 (46), E10925-E10933
- **CRISTIANO S. et al.** *Nature,* 2019, vol. 570, 385-389
- **MOULIERE et al.** *Sci. Transl. Med.,* 2018, vol. 10 (466), eaat4921
- **NEWMAN A. et al.** *Nat. Med.,* 2014, vol. 20 (5), 548-554
- **DE RUBIS G. et al.** *Trends Pharmacol Sci.,* 2019, vol. 40 (3), 172-186
- **PEIYONG JIANG et al.** *Cancer Discov.,* 2020, CD-19-0622
- **PEIYONG JIANG et al.** *Genome Res.,* 2020
- **MARTIN, M. et al.** *EMB.netJournal,* 2011, 17.1
- **LI, H ; DURBIN, R.** *Bioinformatics,* 2009, vol. 25, 1754-1760
- **TARASOV, ARTEM et al.** Sambamba: fast processing of NGS alignment formats. *Bioinformatics,* 2015, vol. 31.12, 2032-2034